Europäisches Patentamt

European Patent Office

Office européen des brevets

(19)

(11) Veröffentlichungsnummer: **0 092 717**
**B1**

# EUROPÄISCHE PATENTSCHRIFT

(12)

(45) Veröffentlichungstag der Patentschrift:
**04.12.85**

(21) Anmeldenummer: **83103447.5**

(22) Anmeldetag: **08.04.83**

(51) Int. Cl.⁴: **G 10 K 11/00, A 61 B 10/00**

(54) **Ultraschall-Applikator.**

(30) Priorität: **26.04.82 DE 3215539**

(43) Veröffentlichungstag der Anmeldung:
**02.11.83 Patentblatt 83/44**

(45) Bekanntmachung des Hinweises auf die Patenterteilung:
**04.12.85 Patentblatt 85/49**

(84) Benannte Vertragsstaaten:
**AT CH DE FR GB LI**

(56) Entgegenhaltungen:
**EP - A - 0 039 045**
**DE - A - 2 950 203**
**DE - A - 3 121 512**
**DE - U - 6 942 159**
**FR - A - 2 450 596**
**US - A - 4 135 826**

(73) Patentinhaber: **Siemens Aktiengesellschaft, Berlin und München Wittelsbacherplatz 2, D-8000 München 2 (DE)**

(72) Erfinder: **Lutz, Harald, Dr., Köhlerstrasse 3, D-8551 Hemhofen (DE)**

BUNDESDRUCKEREI BERLIN

## Beschreibung

Die Erfindung bezieht sich auf einen Ultraschall-Applikator für die Ultraschallabtastung, insbesondere von körperinternen Organen od. dgl., mit einem Ultraschallkopf, der eine Anzahl von Ultraschall-Elementen umfaßt, die von einem länglichen, starren Ultraschallkopf-Gehäuse umschlossen sind, das eine auf das betreffende Organ auflegbare Applikationsfläche besitzt, mit einem länglichen, starren Handgriff und mit einer Halteverbindung zwischen dem Ultraschallkopf und dem Handgriff.

Es sind bereits Ultraschall-Applikatoren dieser Art vorgeschlagen worden, die jedoch alle nur zur Abtastung von körperinternen Organen von der unversehrten Körperoberfläche her ausgebildet sind. Wünschenswert wäre jedoch ein Ultraschall-Applikator, der auch intra-operativ eingesetzt werden kann.

Aus der DE-A-2 950 203 ist bereits ein Endoskop bekannt, das aus einem Okular, einem flexiblen Schlauch, einem steuerbar biegbaren Zwischenstück und einem zylinderförmigen Endstück besteht, wobei der flexible Schlauch einen Lichtleiter und Steuerleitungen und wobei das Endstück einen Ultraschallkopf enthält. Ein solches Endoskop ist für eine intra-operative Behandlung wenig geeignet, da es wegen der empfindlichen Optik kaum ausreichend sterilisiert werden kann. Außerdem gestattet das biegbare Zwischenstück nur ein Arbeiten mit verhältnismäßig geringen Biegewinkeln. Darüber hinaus ist es schwierig, bei begrenztem Operationsfeld das zylinderförmige Endstück so zu plazieren, daß der Ultraschallkopf — vom Operationsfeld aus gesehen — unter dem zu untersuchenden Organ liegt. Die Anwendungsmöglichkeiten sind also recht beschränkt.

Aus der europäischen Patentanmeldung EP-A-0 039 045 ist ebenfalls ein Endoskop bekannt, das an einem manipulierbaren Endstück zwei Ultraschallköpfe trägt. Auch bei diesem Endoskop ist der Manipulationswinkel des Endstücks verhältnismäßig klein. Er dürfte nur wenige Grad betragen. Wegen des im Inneren untergebrachten Manipulationsmechanismus von nicht zu vernachlässigender Ausdehnung kann das Endoskop nur in Operationsfeldern von einer Mindestausdehnung ab verwendet werden.

Aus dem deutschen Gebrauchsmuster DE-U-6 942 159 ist schließlich eine medizinische Ultraschall-Prüfsonde bekannt, die einen Handgriff, eine biegsame Schlauchverbindung und an deren Ende einen Ultraschall-Wandler umfaßt. Ein Abwinkeln des Ultraschall-Wandlers unmittelbar am Ende des Schlauches ist hier nicht möglich, so daß der Untersuchung körperinterner Organe bei einer Operation ebenfalls Grenzen gesetzt sind.

Aufgabe der Erfindung ist es, einen Ultraschall-Applikator zu schaffen, der für den intra-operativen Einsatz bei verschiedenartigen Anwendungsfällen geeignet ist, der leicht handhabbar ist und keinen besonders großen Zugang im Operationsfeld erfordert.

Diese Aufgabe wird erfindungsgemäß dadurch gelöst, daß die Halteverbindung eine Schwenkverbindung ist, die das eine Ende des Ultraschallkopf-Gehäuses unmittelbar mit dem einen Ende des Handgriffs schwenkbar verbindet, daß die Schwenkachse zwischen dem Handgriff und dem Ultraschallkopf-Gehäuse senkrecht zur Längsachse des Handgriffs und parallel zur Ebene der Applikationsfläche verläuft, und daß die Neigung des Ultraschallkopf-Gehäuses bezüglich des Handgriffs von der am Handgriff angreifenden Hand einstellbar ist.

Der schwenkbar gehalterte Ultraschallkopf läßt sich in einfacher Weise von Hand in jede beliebige Applikationslage bringen. Bezüglich des zu untersuchenden körperinternen Organes bedeutet dies, daß der Ultraschall-Applikator beliebig rück-, vorder- oder seitenwandig an das abzutastende Organ angelegt werden kann. Der erfindungsgemäße Ultraschall-Applikator eignet sich also besonders gut zum Einsatz bei Operationen. Der Ultraschallkopf läßt sich praktisch unmittelbar hinter dem Handgriff abwinkeln. Die Applikationsfläche ist voll ausnutzbar, d. h. es sind Untersuchungen in großer Tiefe auch bei kleinem Operationsfeld möglich.

Weitere Vorteile und Einzelheiten der Erfindung ergeben sich aus der nachfolgenden Beschreibung zweier Ausführungsbeispiele anhand der Zeichnung und in Verbindung mit den Unteransprüchen. Es zeigt

Fig. 1 einen Ultraschall-Applikator gemäß der Erfindung in Seitenansicht,

Fig. 2 denselben Ultraschall-Applikator in Vorderansicht,

Fig. 3 Applikationsmöglichkeiten für einen Ultraschall-Applikator gemäß der Erfindung bei intra-operativem Einsatz an einem Körperorgan wie z. B. einer Niere,

Fig. 4 weitere Applikationsmöglichkeiten an einem Organ wie z. B. der Leber.

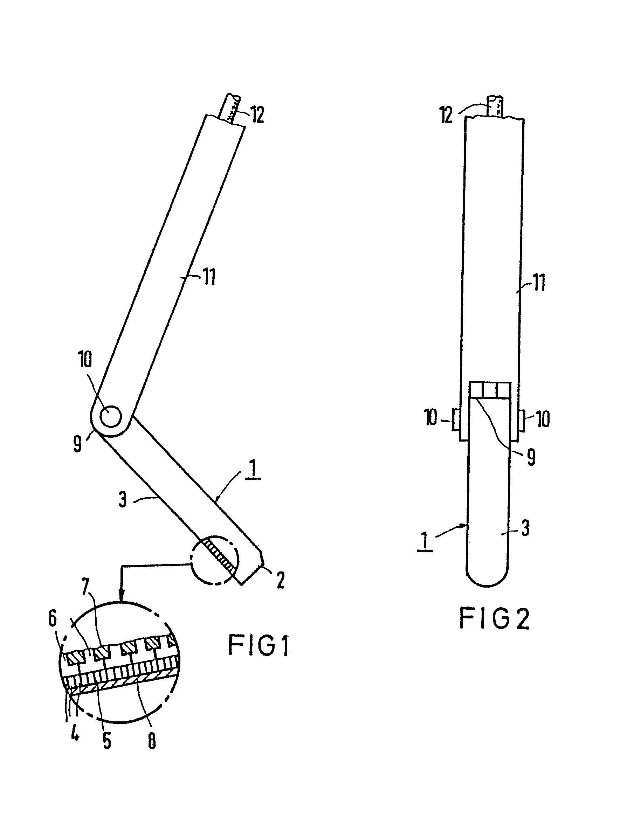

Fig. 1 zeigt einen Ultraschallkopf, der als Ultraschall-Array 1 ausgebildet ist und ein längliches, starres Array-Gehäuse 2 mit einer Applikationsfläche 3 umfaßt. Das Ultraschall-Array 1 besitzt dabei an der Applikationsfläche 3, wie in der Ausschnittsvergrößerung dargestellt ist, eine Vielzahl von Wandlerelementen 4, die vom Gehäuse 2 umschlossen und die im vorliegenden Ausführungsbeispiel feingeteilt sind. Jeweils mehrere dieser feingeteilten Wandlerelemente 4, im vorliegenden Fall insgesamt z. B. vier Wandlerelemente, sind elektrisch zu einer Gruppe 5 (von z. B. insgesamt 48 Gruppen) zusammenkontaktiert. Zur Gruppenkontaktierung dienen Kontaktfahnen 6 eines Kontaktkammes. Mit 7 ist ein Trägerkörper für die Ultraschall-Wandlerelemente 4 bezeichnet. Der Trägerkörper 7, in den die Kontaktfahnen 6 eingebettet sind, besteht beispielsweise aus Epoxydharz mit eingebrachtem oxidiertem Wolframpulver. Mit 8 ist eine Anpassungsschicht für die Wandlerelemente bezeichnet. Die Anpassungsschicht 8 kann aus Epoxydharz bestehen. Das Grundprinzip der Feinteilung von Wandlerelementen ist z. B. in der US-A-4 305 014 näher beschrieben. Die Applikationsfläche 3 kann z. B. eine Länge von 3 Zentimetern besitzen. Die Länge richtet sich nach der Art der in Betracht kommenden Anwendungsfälle.

Gemäß Fig. 1 weist das Ultraschall-Array 1 am Array-Gehäuse 2, und zwar praktisch unmittelbar am Ende 9, ein Drehgelenk 10 auf, mittels dessen das Ultraschall-Array 1 an einem Handgriff 11 mit vorgebbarer Reibung schwenkbar angelenkt ist. Der Handgriff 11 ist verhältnismäßig dünn. Er kann z. B. einen Durchmesser von 10 bis 12 mm haben. Das Array 1 ist in einem Schwenkbereich von vorzugsweise > ±90° bezüglich der Längsachse des Handgriffs 11 verstellbar. Die Verstellung erfolgt manuell von der am Handgriff 11 angreifenden Hand des Operateurs. Nähere Details zur Schwenkbarkeit sind der Fig. 2 entnehmbar, in der auch gezeigt ist, daß das Signalanschlußkabel 12 des Ultraschall-Arrays 1 im Innern des ausgehöhlten Handgriffs 11 geführt ist. Das Signalanschlußkabel 12 ist dabei so gelagert, daß es dem Ultraschall-Array 1 praktisch zugfrei in jede beliebige Schwenklage folgen kann. Das Array 1, der Handgriff 11, das Drehgelenk 10 und das Kabel 12 sind verhältnismäßig einfach zu sterilisieren.

Die Ausführung nach Fig. 1 und 2 hat den Vorteil, daß auch bei kleinem Operationsfeld Organe in tiefen Lagen erreicht werden können. Der Ultraschall-Applikator 1 nach Fig. 1 und 2 eignet sich insbesondere für den intra-operativen Einsatz, obgleich auch ein transkutaner Einsatz möglich ist.

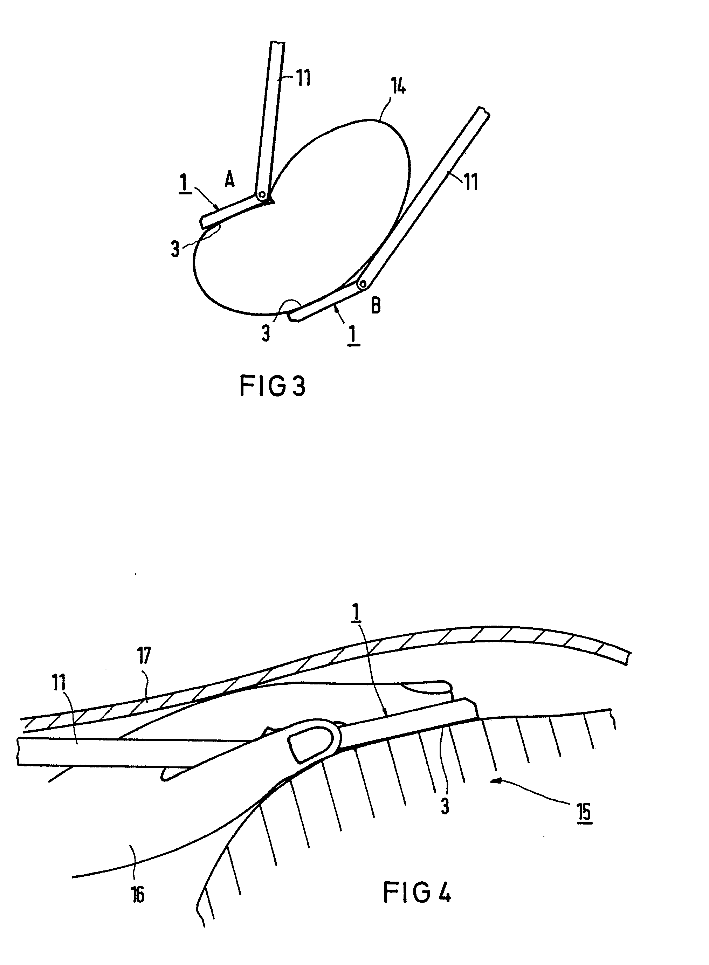

Die Möglichkeit des intra-operativen Einsatzes ist am Beispiel einer Nierenuntersuchung in Fig. 3 dargestellt. Hier ist eine durch die Operation freigelegte Niere mit 14 bezeichnet. Das Ultraschall-Array 1 liegt mit der Applikationsfläche 3 in der Untersuchungsposition A z. B. auf der Vorderwand der Niere 14 an. In der Untersuchungsposition B liegt das Ultraschall-Array 1 mit seiner Applikationsfläche 3 hingegen an der Rückwand der Niere 14. Hieraus wird deutlich, daß das längliche Ultraschallkopf-Gehäuse 2 von einem Neigungswinkel größer 0° in einen Neigungswinkel kleiner 0° geschwenkt werden kann. Der Neigungswinkel ist dabei der zwischen der Applikationsfläche 3 und der Längsachse des Handgriffs 11 liegende Winkel.

Fig. 4 zeigt die intra-operative Anwendung der Erfindung an einer Leber 15. Das Ultraschall-Array 1 wird dazu in der dargestellten Weise mit der Hand 16 umfaßt und nach Einschieben unterhalb des geöffneten Zwerchfells 17 durch Schwenken gegenüber dem Handgriff 11 in eine geeignete Applikationsposition an der Leber 15 gebracht.

Es ist selbstverständlich, daß derartige Untersuchungen nicht nur an einer Niere 14 oder Leber 15, sondern an jedem beliebigen anderen körperinternen Organ oder einem sonstigen inneren Körperteil vorgenommen werden können.

## Patentansprüche

1. Ultraschall-Applikator für die Ultraschallabtastung, insbesondere von körperinternen Organen (14; 15) od. dgl., mit einem Ultraschallkopf (1), der eine Anzahl von Ultraschall-Elementen (4) umfaßt, die von einem länglichen, starren Ultraschallkopf-Gehäuse (2) umschlossen sind, das eine auf das betreffende Organ (14; 15) auflegbare Applikationsfläche (3) besitzt, mit einem länglichen, starren Handgriff (11) und mit einer Halteverbindung zwischen dem Ultraschallkopf (1) und dem Handgriff (11), dadurch gekennzeichnet, daß die Halteverbindung eine Schwenkverbindung (10) ist, die das eine Ende (9) des Ultraschallkopf-Gehäuses (2) unmittelbar mit dem einen Ende des Handgriffs (11) schwenkbar verbindet, daß die Schwenkachse zwischen dem Handgriff (11) und dem Ultraschallkopf-Gehäuse (2) senkrecht zur Längsachse des Handgriffs (11) und parallel zur Ebene der Applikationsfläche (3) verläuft, und daß die Neigung des Ultraschallkopf-Gehäuses (2) bezüglich des Handgriffs (11) von der am Handgriff (11) angreifenden Hand einstellbar ist.

2. Ultraschall-Applikator nach Anspruch 1, dadurch gekennzeichnet, daß der Ultraschallkopf (1) ein Ultraschall-Array ist.

3. Ultraschall-Applikator nach Anspruch 1 oder 2, dadurch gekennzeichnet, daß das Ultraschallkopf-Gehäuse (2) von einem ersten Neigungswinkel kleiner 0° in einen zweiten Neigungswinkel größer 0° schwenkbar ist, wobei der Neigungswinkel jeweils zwischen der Applikationsfläche (3) und der Längsachse des Handgriffs (11) gemessen ist (Fig. 3).

## Claims

1. An ultrasonic applicator for ultrasonic scanning, in particular of internal body organs (14; 15) or the like, having an ultrasonic head (1) which comprises a number of ultrasonic elements (4) surrounded by an oblong, rigid ultrasonic head housing (2) which possesses an application surface (3) that can be placed against the respective organ (14; 15), an oblong, rigid handle (11) and a retaining connection between the ultrasonic head (1) and the handle (11), characterised in that the retaining connection is a swivel connection (10) which directly connects one end (9) of the ultrasonic head housing (2) to one end of the handle (11) in a swivelling fashion, that the swivel axis between the handle (11) and the ultrasonic head housing (2) extends at right angles to the longitudinal axis of the handle (11) and parallel to the plane of the application surface (3), and that the inclination of the ultrasonic head housing (2) in relation to the handle (11) is adjustable by the hand touching the handle (11).

2. An ultrasonic applicator as claimed in Claim 1, characterised in that the ultrasonic head (1) ist an ultrasonic array.

3. An ultrasonic applicator as claimed in Claim 1 or 2, characterised in that the ultrasonic head housing (2) can be swivelled from a first angle of inclination smaller than 0°, into a second angle of inclination greater than 0°, the respective angles of inclination being measured between the application surface (3) and the longitudinal axis of he handle (11) (Fig. 3).

## Revendications

1. Applicateur à ultrasons pour l'examen par balayage aux ultrasons, en particulier d'organes intracorporels (14; 15) ou similaires, comportant une tête à ultrasons (1) qui comporte un certain nombre d'éléments ultrasonores (4) qui sont entourés par un boîtier (2) de tête à ultrasons, rigide et allongée, et possédant une surface d'application (3) susceptible d'être placée sur l'organe (14; 15) concerné, une poignée (11) rigide et allongée et une liaison entre la tête à ultrasons (1) et la poignée (11), caractérisé par le fait que la liaison est une liaison à basculement (10) qui relie de façon articulée une extrémité (9) du boîtier (2) de la tête à ultrasons directement à une extrémité de la poignée (11), que l'axe de basculement entre la poignée (11) et le boîtier (2) de la tête à ultrasons s'étend perpendiculairement à l'axe longitudinal de la poignée (11) et parallèlement au plan de la surface d'application (3), et que l'inclinaison de la poignée (11) est susceptible d'être réglée par la main qui attaque le poignée (11).

2. Applicateur à ultrasons selon la revendication 1, caractérisé par le fait que la tête à ultrasons (1) est constituée par un système d'éléments ultrasonores.

3. Applicateur à ultrasons selon la revendication 1 ou 2, caractérisé par le fait que le boîtier (2) de la tête à ultrasons est susceptible d'être basculé d'un premier angle d'inclinaison inférieur à 0° à un second angle d'inclinaison supérieur à 0°, l'angle d'inclinaison étant mesuré entre la surface d'application (3) et l'axe longitudinal de la poignée (figure 3).

FIG 1

FIG 2

FIG 3

FIG 4